# EUROPEAN PATENT APPLICATION

(11) **EP 4 692 051 A1**
(43) Date of publication of application: **11.02.2026**
(21) Application number: 24784949.0
(22) Date of filing: 04.04.2024
(51) Int. Cl.: C07C 273/04, B01J 10/00, B01J 19/00, B01J 19/26, C07C 275/00

(54) **UREA SYNTHESIS APPARATUS, UREA SYNTHESIS METHOD, AND METHOD FOR IMPROVING EXISTING UREA SYNTHESIS APPARATUS**

(30) Priority: 07.04.2023 JP 2023062999
(71) Applicant: Toyo Engineering Corporation, Chiba-shi, Chiba 261-8601 (JP)
(72) Inventor: YANAGAWA, Takahiro, Chiba-shi, Chiba 261-8601 (JP); KOJIMA, Yasuhiko, Chiba-shi, Chiba 261-8601 (JP); KUNII, Hiroo, Chiba-shi, Chiba 261-8601 (JP)
(74) Representative: Gille Hrabal Partnerschaftsgesellschaft mbB Patentanwälte
(86) International application number: PCT/JP2024/013911
(87) International publication number: WO 2024/210167

(57) **Abstract**

Provided is a urea synthesis apparatus enabling the facilitation of apparatus maintenance, the facilitation of apparatus design or fabrication, and/or the acceleration of a urea reaction. The present invention is a urea synthesis apparatus for reacting ammonia and carbon dioxide to produce urea, and includes a first urea synthesis reactor (R1), a second urea synthesis reactor (R2), a condenser (C), a stripper (S) and means (E) for supplying a first urea synthesis liquid. The condenser (C) is a device for condensing a mixed gas into an absorbing medium to obtain a condensed liquid by allowing a process fluid to pass through a tube (Ct) and cooling it with a cooling medium passing through a shell (Cs) side, the condenser (C) being a device separate from the first urea synthesis reactor (R1) and the second urea synthesis reactor (R2), the first urea synthesis reactor (R1) is a device for obtaining the first urea synthesis liquid from the condensed liquid obtained in the condenser (C), and the second urea synthesis reactor (R2) is a device for obtaining a second urea synthesis liquid from the first urea synthesis liquid.

## Description

### Field of the Invention

The present invention relates to a urea synthesis apparatus enabling the facilitation of apparatus maintenance, the facilitation of apparatus design or fabrication, and/or the acceleration of a urea reaction.

### Background of the Invention

In a urea synthesis process, usually, carbon dioxide and ammonia are reacted at a high temperature and under a high pressure to obtain a urea synthesis liquid. This urea synthesis liquid contains not only urea produced by the synthesis reaction, but also unreacted ammonia, unreacted carbon dioxide, and ammonium carbamate, an intermediate. Further, these unreacted components and intermediate are removed from the urea synthesis liquid to obtain a urea product.

The unreacted components and intermediate removed from the urea synthesis liquid are usually recycled as raw materials for urea synthesis. For example, a mixed gas (a gas containing unreacted ammonia and unreacted carbon dioxide) separated from the urea synthesis liquid in a stripper is condensed into an absorbing medium in a condenser to obtain a condensed liquid, and the obtained condensed liquid is recycled as a raw material for urea synthesis. As this absorbing medium, for example, an ammonium carbamate liquid (recycle carbamate liquid) separated and recovered from the urea synthesis liquid in a subsequent step can be used.

However, ammonium carbamate is highly corrosive to metal, and thus, a portion made in contact with the absorbing medium (recycle carbamate liquid) in the condenser needs to be made of a highly corrosion-resistant metal. Further, a welded portion of the portion made in contact with the absorbing medium needs to be protected from crevice corrosion by using full penetration welding without a crevice (for example, internal bore welding). Further, periodic inspections for checking the presence or absence of occurrence of malfunctions such as corrosion and others are also required.

On the other hand, as typical examples of the condenser for condensing the mixed gas into the absorbing medium, condensers having a shell-and-tube structure, such as a fixed-tubesheet condenser, a U-tube condenser and others are known. The fixed-tubesheet condenser is a condenser in which tubesheets supporting both ends of tubes are fixed on both sides of a shell. Due to that structure, it is relatively easy to inspect or clean the interior of the tubes, but is difficult to inspect or clean a shell side. Accordingly, in the fixed-tubesheet condenser, a process fluid containing the absorbing medium (recycle carbamate liquid) which is highly corrosive needs to be allowed to pass through the tubes. On the other hand, the U-tube condenser is a condenser in which U-shaped tubes are used and a tubesheet supporting these is fixed on one side. Due to that structure, it is relatively easy to inspect or clean the interior of the tubes, and on a shell side, it is relatively easy to inspect or clean a tube locating on outer side of the tube bundle (tube group), but is relatively difficult to inspect or clean a tube locating on inner side of the tube bundle.

Further, in the condenser, not only condensation, but also a urea synthesis reaction takes place. Further, in the U-tube condenser, a reaction volume (a volume of the process fluid retained in the condenser per unit time) is larger when the process fluid is allowed to pass through the shell side than when it is allowed to flow through the tube side. Accordingly, the U-tube condenser in which the process fluid is allowed to pass through the shell side (hereinafter referred to as a U-tube submerged condenser) is superior to the fixed-tubesheet condenser in terms of the acceleration of the urea synthesis reaction within the condenser.

However, when the process fluid is allowed to pass through the shell side in the U-tube submerged condenser, due to its structure, the tubes and the tubesheet need to be welded by full penetration welding (for example, internal bore welding) to prevent crevice corrosion. When such special welding is employed, the efficiency of device fabrication is reduced, and welding work costs are also increased. Further, inspection or cleaning of the shell side requires a relatively large space for maintenance between the tube bundle and the interior of the shell. This is particularly an obstacle to downsizing of a plant. These are problems in the U-tube submerged condenser.

WO-A 2006-118071 describes a urea synthesis apparatus with an integrated synthesis reactor and condenser. This condenser is a U-tube submerged condenser, in which a process fluid passes through a shell side. On the other hand, a cooling medium passes through a tube, and cools the process fluid on the shell side, and a mixed gas is condensed into an absorbing medium under this cooling to obtain a condensed liquid. Further, in this apparatus, the heavy condenser is integrally installed at a lower position than the synthesis reactor, and thus, it is superior in terms of easy installation or fixing of the equipment compared to when the condenser is integrally installed at a higher position than the synthesis reactor.

However, this U-tube submerged condenser has some problems as described above. Moreover, in downsizing of a plant, it is difficult for an integrated apparatus of synthesis reactor and U-tube submerged condenser as described in WO-A 2006-118071 to downsize not only the condenser but also the synthesis reactor integrated therewith, or if the synthesis reactor is adapted to a required volume, its length becomes too short for its diameter which is dependent on requirement of maintenance space for condenser, and optimum design is impossible. In other words, the apparatus of WO-A 2006-118071 has room for improvement in these points.

JP-A 2006-102590 describes a reactor comprising a first plate member made of a high corrosion-resistant metal and a second plate member made of a low corrosion-resistant metal, wherein while heat exchange tubes (tubes) are welded to the first plate member by internal bore welding, the second plate member is provided detachably. This reactor is superior in that, as the second plate member is detachable, it is easy to weld portions where the heat exchange tubes are attached to the first plate member or to inspect the device. However, this second plate member is very heavy, and attachment or removal of this requires a large crane or lifting structure, and also requires a large space for attachment or removal work.

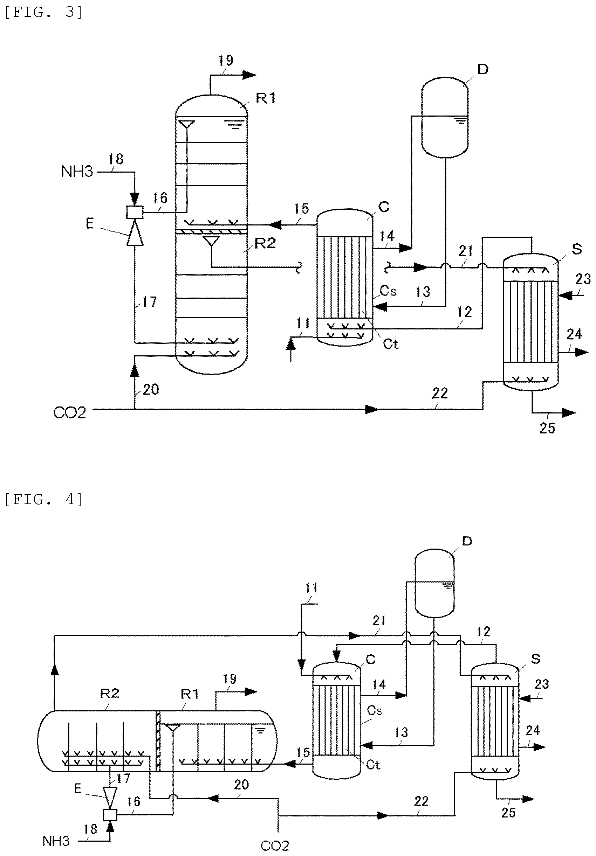

WO-A 01-72700 describes an installation comprising two reactor sections (synthesis reactors). Particularly, Figure 3 thereof describes an installation comprising a combined upper synthesis reactor, condenser and lower synthesis reactor, and Figure 4B describes an installation comprising a combined upper synthesis reactor and lower synthesis reactor and a horizontally placed poolcondenser.

This horizontally placed poolcondenser described in Figure 4B is a U-tube submerged condenser, and thus has some problems as described earlier. Further, the circulation of a synthesis liquid (circulation by which a liquid, a gas or a liquid-gas mixture is allowed to flow through the condenser, the upper synthesis reactor, the lower synthesis reactor, a stripper and the condenser in sequence) is performed by a pressure of each device and gravity (a difference in height), and thus, the circulation flow is susceptible to the pressure of each device, and a circulation flow rate is difficult to control. Moreover, relative height of location of each device is subject to constraints, and a plant height becomes relatively high.

On the other hand, the condenser described in Figure 3 is not explained specifically, but is considered to be a fixed tubesheet condenser from representation in the figure. Further, WO-A 01-72700 explains that cooling medium in a heat exchanger (condenser) may sometimes also be allowed to pass through a shell side. However, as the condenser described in Figure 3 is combined with the upper synthesis reactor and the lower synthesis reactor, a worker needs to enter the interior from the cylindrical portion above or below the condenser when performing inspection of welding of tubes and a tubesheet of the condenser, and that work involves difficulty. Further, as a shell diameter of the condenser needs to be determined to suit the synthesis reactor, optimum design of the shell diameter of the condenser is difficult. Further, as the synthesis reactors are connected above and below the condenser, a height of the device becomes very high.

### Summary of the Invention

An object of the present invention is to solve the problems of each conventional apparatus explained above, and provide a urea synthesis apparatus enabling the facilitation of apparatus maintenance, the facilitation of apparatus design or fabrication, and/or the acceleration of a urea reaction.

As a result of making intensive studies to achieve the above object, the present inventors found out that it is very effective to provide a condenser as a device separate from a reactor and allow a process fluid to pass through a tube, and further, to provide at least two reactors, and reached the completion of the present invention.

In other words, the present invention is a urea synthesis apparatus for reacting ammonia and carbon dioxide to produce urea, including,
a first urea synthesis reactor (R1), a second urea synthesis reactor (R2), a condenser (C), a stripper (S) and means (E) for supplying a first urea synthesis liquid,
wherein the condenser (C) is a device including a tube (Ct) inside and for condensing a mixed gas separated in the stripper (S) into an absorbing medium to obtain a condensed liquid by allowing a process fluid containing the absorbing medium and the mixed gas to pass through the tube (Ct) and cooling the process fluid with a cooling medium passing through a shell (Cs) side, the condenser (C) being a device separate from the first urea synthesis reactor (R1) and the second urea synthesis reactor (R2),
wherein the first urea synthesis reactor (R1) is a device for reacting at least some components of components in the condensed liquid obtained in the condenser (C) to obtain the first urea synthesis liquid containing urea, unreacted ammonia, unreacted carbon dioxide and water,
wherein the means (E) for supplying the first urea synthesis liquid is means for supplying the first urea synthesis liquid obtained in the first urea synthesis reactor (R1) to the second urea synthesis reactor (R2),
wherein the second urea synthesis reactor (R2) is a device for reacting at least some components of the components in the first urea synthesis liquid obtained in the first urea synthesis reactor (R1), at least part of the raw material ammonia and at least part of the raw material carbon dioxide to obtain a second urea synthesis liquid containing urea, unreacted ammonia, unreacted carbon dioxide and water, and
wherein the stripper (S) is a device for stripping the second urea synthesis liquid obtained in the second urea synthesis reactor (R2) using at least part of the raw material carbon dioxide to separate the mixed gas containing unreacted ammonia and unreacted carbon dioxide from the second urea synthesis liquid.

Further, the present invention is a urea synthesis method for obtaining a urea synthesis liquid using the above urea synthesis apparatus of the present invention.

Further, the present invention is a method for improving an existing urea synthesis apparatus, the existing urea synthesis apparatus including a urea synthesis reactor,
wherein the improving method includes:
adding to the existing urea synthesis apparatus at least a first urea synthesis reactor (R1) and means (E) for supplying a first urea synthesis liquid; and
when the existing urea synthesis apparatus does not include one or either of a condenser (C) and a stripper (S), adding the condenser (C) and/or the stripper (S) not included, and
wherein the condenser (C) is a device including a tube (Ct) inside and for condensing a mixed gas separated in the stripper (S) into an absorbing medium to obtain a condensed liquid by allowing a process fluid containing the absorbing medium and the mixed gas to pass through the tube (Ct) and cooling the process fluid with a cooling medium passing through a shell (Cs) side, the condenser (C) being a device separate from the first urea synthesis reactor (R1) and a second urea synthesis reactor (R2),
wherein the first urea synthesis reactor (R1) is a device for reacting at least part of components in the condensed liquid obtained in the condenser (C) to obtain the first urea synthesis liquid containing urea, unreacted ammonia, unreacted carbon dioxide and water,
wherein the means (E) for supplying the first urea synthesis liquid is means for supplying the first urea synthesis liquid obtained in the first urea synthesis reactor (R1) to the second urea synthesis reactor (R2),
wherein the second urea synthesis reactor (R2) is at least part of the urea synthesis reactor included in the existing urea synthesis apparatus, and is a device for reacting at least some components in the first urea synthesis liquid obtained in the first urea synthesis reactor (R1), at least part of raw material ammonia and at least part of raw material carbon dioxide to obtain a second urea synthesis liquid containing urea, unreacted ammonia, unreacted carbon dioxide and water, and
wherein the stripper (S) is a device for stripping the second urea synthesis liquid obtained in the second urea synthesis reactor (R2) using at least part of the raw material carbon dioxide to separate the mixed gas containing unreacted ammonia and unreacted carbon dioxide from the second urea synthesis liquid.

In the urea synthesis apparatus of the present invention, the process fluid is allowed to pass through the tube (Ct) of the condenser (C), and the cooling medium is allowed to pass through the shell (Cs) side, and thus, special welding (for example, internal bore welding) is unnecessary, and maintenance for inspection or cleaning can be performed easily.

Further, in the urea synthesis apparatus of the present invention, the condenser (C) is a device separate from the first urea synthesis reactor (R1) and the second urea synthesis reactor (R2), and thus, inspection of the interior of the condenser (C) is easy and optimum design of a shell diameter of the condenser (C) is easy, and heights of the synthesis reactors can also be reduced, as compared to an apparatus in which it is integrated therewith.

Further, in the urea synthesis apparatus of the present invention, a reaction volume of the condenser (C) is relatively small, but the two urea synthesis reactors (R1) and (R2) are included, and thus, the apparatus can accelerate the urea reaction while suppressing an uncondensed gas by optimizing reaction conditions of each synthesis portion.

### Brief Description of the Drawings

[FIG. 1] A process flow diagram showing a first embodiment of the apparatus of the present invention.
[FIG. 2] A process flow diagram showing a second embodiment of the apparatus of the present invention.
[FIG. 3] A process flow diagram showing a third embodiment of the apparatus of the present invention.
[FIG. 4] A process flow diagram showing a fourth embodiment of the apparatus of the present invention.

### Embodiments of the Invention

The urea synthesis apparatus of the present invention is an apparatus for reacting ammonia and carbon dioxide to produce urea, and includes a first urea synthesis reactor (R1), a second urea synthesis reactor (R2), a condenser (C), a stripper (S) and means (E) for supplying a first urea synthesis liquid. Hereinafter, those are each explained.

### <Condenser (C)>

The condenser (C) is a device including a tube (Ct) inside and for condensing a mixed gas separated in the stripper (S) into an absorbing medium to obtain a condensed liquid by allowing a process fluid containing the absorbing medium and the mixed gas to pass through the tube (Ct) and cooling the process fluid with a cooling medium passing through a shell (Cs) side. Accordingly, it does not require special welding (for example, internal bore welding), and can reduce a space for maintenance for inspection or cleaning. Further, if the corrosive process fluid at a high temperature and a high pressure is allowed to flow through the shell (Cs) side, both the tube (Ct) and the shell (Cs) need to be formed of a corrosion-resistant material, but the present invention in which the process fluid is allowed to pass through the tube (Ct) makes it possible to use a corrosion-resistant material only for the tube. The corrosion-resistant material is, for example, a high-chromium austenitic steel, a duplex alloy, titanium or 316L austenitic steel or the like. As the corrosive process fluid at a high temperature and a high pressure is allowed to flow through the tube (Ct), design constraints on the shell (Cs) can be reduced, and the shell (Cs) can be formed of an inexpensive and highly reliable material, for example, carbon steel, a low alloy steel or the like.

A temperature and a pressure for condensation in the condenser (C) are preferably 160 to 200°C and preferably 13 to 25 MPa, respectively.

The condenser (C) is a device separate from the first urea synthesis reactor (R1) and the second urea synthesis reactor (R2). Accordingly, inspection of the interior of the condenser (C) is easy, and optimum design of a shell diameter of the condenser (C) is easy.

A fixed-tubesheet condenser is preferably used as the condenser (C) in terms of no necessity of special welding and easy inspection or cleaning. Further, the tube (Ct) is preferably a straight tube. However, the present invention is not limited thereto. For example, a condenser which has a U-tube type head at a rear end of the head can also be used as the condenser (C). It may be configured in such a manner that a U-shaped tube is used as the tube (Ct) and the tube (Ct) is supported by a tubesheet for fixing one side of the U-shape.

### <First urea synthesis reactor (R1)>

The first urea synthesis reactor (R1) is a device for reacting at least some components of components in the condensed liquid obtained in the condenser (C) to obtain the first urea synthesis liquid containing urea, unreacted ammonia, unreacted carbon dioxide and water. This first urea synthesis reactor (R1) complements the function of urea synthesis in the condenser (C), and the apparatus can accelerate the urea reaction while suppressing an uncondensed gas by optimizing reaction conditions of this first urea synthesis reactor (R1) and the second urea synthesis reactor (R2) described later.

Preferable reaction conditions in the first urea synthesis reactor (R1) are different from those in the second urea synthesis reactor (R2) described later. Specifically, a temperature and a pressure for urea synthesis in the first urea synthesis reactor (R1) are preferably 160 to 200°C and preferably 13 to 25 MPa, respectively. Further, N/C is preferably 2.5 to 4.0.

A structure of a synthesis reactor used as the first urea synthesis reactor (R1) is not particularly limited. A synthesis reactor of a publicly-known structure used for urea synthesis can be used.

### <Means (E) for supplying first urea synthesis liquid>

The means (E) for supplying the first urea synthesis liquid is means for supplying the first urea synthesis liquid obtained in the first urea synthesis reactor (R1) to the second urea synthesis reactor (R2).

This means (E) for supplying the first urea synthesis liquid preferably includes an ejector. Further, this ejector is preferably driven by the raw material ammonia as a driving source. In this case, the means (E) for supplying the first urea synthesis liquid also functions as circulating means, and it becomes easy to control a circulation flow rate of the process fluid flowing through the devices constituting the synthesis apparatus (step) (the condenser, the first synthesis reactor, the second synthesis reactor and the stripper) in sequence, and moreover, it is also possible to install the first urea synthesis reactor (R1) and/or the second urea synthesis reactor (R2) on the ground. However, the present invention is not limited thereto. For example, a pump can also be used as the means (E) for supplying the first urea synthesis liquid.

### <Second urea synthesis reactor (R2)>

The second urea synthesis reactor (R2) is a device for reacting at least some components of the components in the first urea synthesis liquid obtained in the first urea synthesis reactor (R1), at least part of the raw material ammonia and at least part of the raw material carbon dioxide to obtain a second urea synthesis liquid containing urea, unreacted ammonia, unreacted carbon dioxide and water.

Preferable temperature and pressure in the second urea synthesis reactor (R2) are different from the aforementioned preferable temperature and pressure in the first urea synthesis reactor (R1). Specifically, a temperature and a pressure for urea synthesis in the second urea synthesis reactor (R2) are preferably 170 to 210°C and preferably 13 to 25 MPa, respectively. Further, N/C is preferably 3 to 4.5.

A structure of a synthesis reactor used as the second urea synthesis reactor (R2) is not particularly limited. A synthesis reactor of a publicly-known structure used for urea synthesis can be used.

Further, the first urea synthesis reactor (R1) and the second urea synthesis reactor (R2) may be devices separate from one another, or may form an integrated device in a configuration of a single partitioned vessel. Further, it may be a vertical device or a horizontal device.

### <Stripper (S)>

The stripper (S) is a device for stripping the second urea synthesis liquid obtained in the second urea synthesis reactor (R2) using at least part of the raw material carbon dioxide to separate the mixed gas containing unreacted ammonia and unreacted carbon dioxide from the second urea synthesis liquid.

A temperature or pressure in the stripper (S) is not particularly limited. Any publicly-known stripping temperature or pressure used for urea synthesis may be employed. Further, a structure of the stripper (S) is not particularly limited, either. A stripper of a publicly-known structure used for urea synthesis can be used.

### <Urea synthesis method>

The urea synthesis method of the present invention is a method for obtaining a urea synthesis liquid using the urea synthesis apparatus of the present invention explained above. This urea synthesis method enables the facilitation of apparatus maintenance, the facilitation of apparatus design or fabrication, and/or the acceleration of the urea reaction as mentioned above.

### <Method for improving existing urea synthesis apparatus>

It is also possible to improve an existing urea production apparatus to form the urea synthesis apparatus of the present invention by adding thereto at least the first urea synthesis reactor (R1) and the means (E) for supplying the first urea synthesis liquid. This improving method enables the facilitation of maintenance of the existing urea synthesis apparatus, the facilitation of apparatus design or fabrication, and/or the acceleration of the urea reaction.

Further, this improving method allows at least part of a urea synthesis reactor included in the existing urea synthesis apparatus to function as the second urea synthesis reactor (R2) in the present invention. When the existing urea synthesis apparatus includes only one urea synthesis reactor, the second urea synthesis reactor (R2) in this improving method is the urea synthesis reactor included in the existing urea synthesis apparatus. On the other hand, when the existing urea synthesis apparatus includes two or more urea synthesis reactors, for example, when a tubular device partitioned internally is allowed to function as the two or more urea synthesis reactors, or the like, part or all of the two or more urea synthesis reactors may be the second urea synthesis reactor (R2).

Further, when the existing urea synthesis apparatus does not include one or either of a condenser (C) and a stripper (S), the condenser (C) and/or the stripper (S) may be added. For example, when the existing urea synthesis apparatus is a urea synthesis apparatus of a solution-recycle type (non-stripping type), the condenser (C) and the stripper (S) are also added. Further, for example, when the existing urea synthesis apparatus is a urea synthesis apparatus of an ammonia-stripping type, a stripper thereof (a stripper to which carbon dioxide is not supplied) is removed, and instead, the stripper (S) in the present invention is also added.

Hereinafter, embodiments of the apparatus of the present invention are explained using drawings.

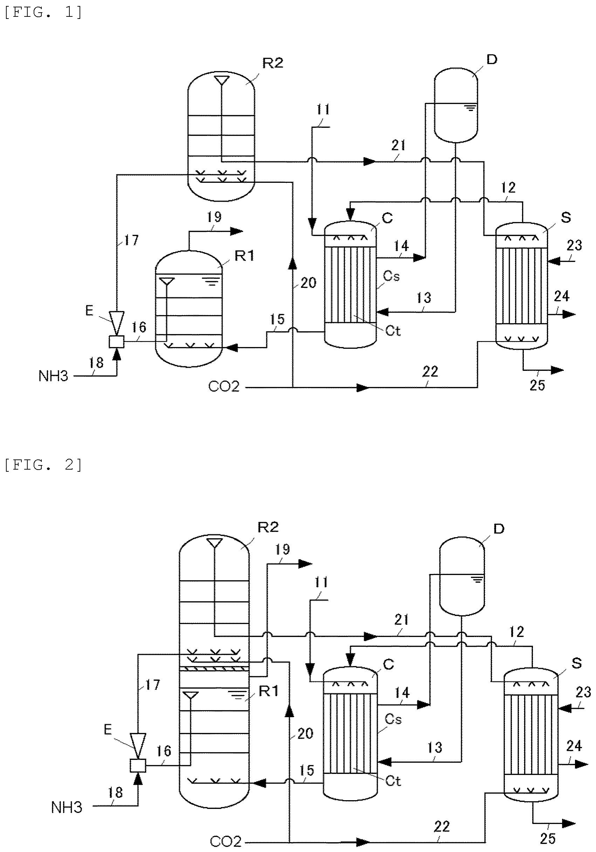

FIG. 1 is a process flow diagram showing a first embodiment of the apparatus of the present invention. In this first embodiment, the first urea synthesis reactor (R1) and the second urea synthesis reactor (R2) are devices separate from one another. As compared to other embodiments, this first embodiment is advantageous in that, as the urea synthesis reactors are separate from one another, placement or device sizing can be flexibly designed. Further, it is also suitable for improvements using an existing urea synthesis reactor.

In FIG. 1, an absorbing medium is supplied to an upper side of the condenser (C) via a line 11, and a mixed gas separated in the stripper (S) is supplied to an upper side of the condenser (C) via a line 12. The condenser (C) includes the tube (Ct) inside, and a process fluid (the absorbing medium and the mixed gas) is allowed to pass through the tube (Ct). A distributing device is provided within a channel of the condenser (C), and is configured to enable the absorbing medium and the mixed gas to pass through each tube (Ct) uniformly. On the other hand, a cooling medium is stored in a steam drum (D), and this cooling medium is supplied to a shell (Cs) side of the condenser (C) via a line 13, recovered via a line 14, and allowed to circulate through this path. The process fluid passing through the tube (Ct) is cooled by this cooling medium. Further, the mixed gas is condensed into the absorbing medium by this cooling to obtain a condensed liquid.

In the first urea synthesis reactor (R1) in FIG. 1, the condensed liquid obtained in the condenser (C) is supplied from a lower side of the condenser (C) via a line 15. Further, at least part of components in the condensed liquid are reacted to obtain a first urea synthesis liquid. This first urea synthesis liquid is supplied via a line 16 to an ejector, the means (E) for supplying the first urea synthesis liquid, and this ejector supplies it to the second urea synthesis reactor (R2) via a line 17. A driving source of this ejector is raw material ammonia supplied from a line 18. On the other hand, the condensed liquid supplied via the line 15 also contains some mixed gas not condensed in the condenser (C). This some mixed gas is separated in the first urea synthesis reactor (R1) and supplied to a device in a subsequent step via a line 19. A scrubber may be installed at a gas outlet of the first urea synthesis reactor (R1) to recover part of the separated mixed gas.

In the second urea synthesis reactor (R2) in FIG. 1, at least some components in the first urea synthesis liquid, at least part of the raw material ammonia added and mixed to the first urea synthesis liquid as a driving source of the ejector, and at least part of raw material carbon dioxide supplied via a line 20 are reacted to obtain a second urea synthesis liquid.

In the stripper (S) in FIG. 1, the second urea synthesis liquid obtained in the second urea synthesis reactor (R2) is supplied via a line 21. Further, this second urea synthesis liquid is stripped using at least part of the raw material carbon dioxide supplied via a line 22 to separate the mixed gas containing unreacted ammonia and unreacted carbon dioxide from the second urea synthesis liquid. Heating in this stripping is performed by steam supplied and discharged via lines 23 and 24. The separated mixed gas is supplied to the condenser (C) via the line 12 as described above. On the other hand, a urea synthesis liquid after the separation of the mixed gas is supplied via a line 25 to a device in a subsequent step (for example, a purification device).

FIG. 2 is a process flow diagram showing a second embodiment of the apparatus of the present invention. In this second embodiment, the first urea synthesis reactor (R1) and the second urea synthesis reactor (R2) form an integrated device in a configuration of a single partitioned vessel, and form a vertical device with the first urea synthesis reactor (R1) located on a lower side and the second urea synthesis reactor (R2) located on an upper side. As compared to other embodiments, this second embodiment is advantageous in that reduction of pressure-resistant members or a reduction of a device weight is achieved, and an area necessary for placement can be reduced.

Each device in FIG. 2 is the same as each device shown in FIG. 1 except that the first urea synthesis reactor (R1) and the second urea synthesis reactor (R2) are changed to the integrated device. Further, each line is also the same as each line shown in FIG. 1.

FIG. 3 is a process flow diagram showing a third embodiment of the apparatus of the present invention. In this third embodiment, the first urea synthesis reactor (R1) and the second urea synthesis reactor (R2) form an integrated device in a configuration of a single partitioned vessel, and form a vertical device with the first urea synthesis reactor (R1) located on an upper side and the second urea synthesis reactor (R2) located on a lower side.

Each device in FIG. 3 is the same as each device shown in FIG. 2 except that the vertical locations of the first urea synthesis reactor (R1) and the second urea synthesis reactor (R2) are reversed, and the gas from the stripper and the absorbing medium are supplied to below the tube (Ct) of the condenser, and allowed to flow through the tube (Ct) upwardly from below. Each line is the same as each line shown in FIG. 2 except that, from FIG. 2, the line 11 for supplying the absorbing medium to the upper side of the condenser (C) is changed to a line for supplying it to a lower side, the line 12 for supplying the mixed gas to the upper side of the condenser (C) is changed to a line for supplying it to a lower side, and the line 15 for discharging the condensed liquid from the lower side of the condenser (C) is changed to a line for discharging it from an upper side. These changes of the lines are made for adapting them to the change of the locations of the first urea synthesis reactor (R1) and the second urea synthesis reactor (R2). As compared to other embodiments, this third embodiment is advantageous in that, as the channel portion and the tube (Ct) of the condenser are filled with the liquid and the gas, a retention time during which they remain within the condenser (reaction time) is increased, and urea synthesis from the absorbed components is accelerated.

FIG. 4 is a process flow diagram showing a fourth embodiment of the apparatus of the present invention. In this fourth embodiment, the first urea synthesis reactor (R1) and the second urea synthesis reactor (R2) form an integrated device in a configuration of a single partitioned vessel, and form a horizontal device with the first urea synthesis reactor (R1) and the second urea synthesis reactor (R2) serially connected to one another. As compared to other embodiments, this fourth embodiment is advantageous in that a plant height can be reduced.

Each device in FIG. 4 is the same as each device shown in FIG. 2 except that the first urea synthesis reactor (R1) and the second urea synthesis reactor (R2) are changed to the horizontal device in which they are serially connected to one another. Further, each line is also the same as each line shown in FIG. 2.

### Industrial Applicability

The urea synthesis apparatus of the present invention enables the facilitation of apparatus maintenance, the facilitation of apparatus design or fabrication, and/or the acceleration of the urea reaction, and thus can be applied very suitably to a urea production plant. Further, it is also useful for improving an existing urea synthesis apparatus.

### Reference Signs List

- R1: first urea synthesis reactor
- R2: second urea synthesis reactor
- C: condenser
- Ct: tube
- Cs: shell
- S: stripper
- E: means for supplying first urea synthesis liquid
- D: steam drum
- 11-25: line

## Claims

1. A urea synthesis apparatus for reacting ammonia and carbon dioxide to produce urea, comprising,
a first urea synthesis reactor (R1), a second urea synthesis reactor (R2), a condenser (C), a stripper (S) and means (E) for supplying a first urea synthesis liquid,
wherein the condenser (C) is a device including a tube (Ct) inside and for condensing a mixed gas separated in the stripper (S) into an absorbing medium to obtain a condensed liquid by allowing a process fluid containing the absorbing medium and the mixed gas to pass through the tube (Ct) and cooling the process fluid with a cooling medium passing through a shell (Cs) side, the condenser (C) being a device separate from the first urea synthesis reactor (R1) and the second urea synthesis reactor (R2),
wherein the first urea synthesis reactor (R1) is a device for reacting at least some components of components in the condensed liquid obtained in the condenser (C) to obtain the first urea synthesis liquid containing urea, unreacted ammonia, unreacted carbon dioxide and water,
wherein the means (E) for supplying the first urea synthesis liquid is means for supplying the first urea synthesis liquid obtained in the first urea synthesis reactor (R1) to the second urea synthesis reactor (R2),
wherein the second urea synthesis reactor (R2) is a device for reacting at least some components of the components in the first urea synthesis liquid obtained in the first urea synthesis reactor (R1), at least part of the raw material ammonia and at least part of the raw material carbon dioxide to obtain a second urea synthesis liquid containing urea, unreacted ammonia, unreacted carbon dioxide and water, and
wherein the stripper (S) is a device for stripping the second urea synthesis liquid obtained in the second urea synthesis reactor (R2) using at least part of the raw material carbon dioxide to separate the mixed gas containing unreacted ammonia and unreacted carbon dioxide from the second urea synthesis liquid.

2. The apparatus according to claim 1, wherein the means (E) for supplying the first urea synthesis liquid comprises an ejector, and the ejector is driven by the raw material ammonia as a driving source.

3. The apparatus according to claim 1, wherein the first urea synthesis reactor (R1) and the second urea synthesis reactor (R2) are devices separate from one another.

4. The apparatus according to claim 1, wherein the first urea synthesis reactor (R1) and the second urea synthesis reactor (R2) form an integrated device in a configuration of a single partitioned vessel.

5. The apparatus according to claim 1, wherein the mixed gas from the stripper (S) and the absorbing medium are supplied from below the condenser (C) and allowed to flow upwardly.

6. A urea synthesis method for obtaining a urea synthesis liquid using the urea synthesis apparatus according to claim 1.

7. A method for improving an existing urea synthesis apparatus, the existing urea synthesis apparatus including a urea synthesis reactor,
wherein the improving method comprises:
adding to the existing urea synthesis apparatus at least a first urea synthesis reactor (R1) and means (E) for supplying a first urea synthesis liquid; and
when the existing urea synthesis apparatus does not include one or either of a condenser (C) and a stripper (S), adding the condenser (C) and/or the stripper (S) not included, and
wherein the condenser (C) is a device including a tube (Ct) inside and for condensing a mixed gas separated in the stripper (S) into an absorbing medium to obtain a condensed liquid by allowing a process fluid containing the absorbing medium and the mixed gas to pass through the tube (Ct) and cooling the process fluid with a cooling medium passing through a shell (Cs) side, the condenser (C) being a device separate from the first urea synthesis reactor (R1) and a second urea synthesis reactor (R2),
wherein the first urea synthesis reactor (R1) is a device for reacting at least part of components in the condensed liquid obtained in the condenser (C) to obtain the first urea synthesis liquid containing urea, unreacted ammonia, unreacted carbon dioxide and water,
wherein the means (E) for supplying the first urea synthesis liquid is means for supplying the first urea synthesis liquid obtained in the first urea synthesis reactor (R1) to the second urea synthesis reactor (R2),
wherein the second urea synthesis reactor (R2) is at least part of the urea synthesis reactor included in the existing urea synthesis apparatus, and is a device for reacting at least some components in the first urea synthesis liquid obtained in the first urea synthesis reactor (R1), at least part of raw material ammonia and at least part of raw material carbon dioxide to obtain a second urea synthesis liquid containing urea, unreacted ammonia, unreacted carbon dioxide and water, and
wherein the stripper (S) is a device for stripping the second urea synthesis liquid obtained in the second urea synthesis reactor (R2) using at least part of the raw material carbon dioxide to separate the mixed gas containing unreacted ammonia and unreacted carbon dioxide from the second urea synthesis liquid.

8. The improving method according to claim 7, wherein the second urea synthesis reactor (R2) is a urea synthesis reactor included in the existing urea synthesis apparatus.

9. The improving method according to claim 7, wherein the mixed gas from the stripper (S) and the absorbing medium are supplied from below the condenser (C) and allowed to flow upwardly.
